# FASCICULE DE BREVET EUROPEEN

(11) **EP 0 423 301 B1**
(45) Date de publication et mention de la délivrance du brevet: **26.01.1994**
(21) Numéro de dépôt: 90907150.8
(22) Date de dépôt: 23.04.1990
(51) Int. Cl.: C07K 7/02, A61K 37/02, G01N 33/564

(54) **PEPTIDES SYNTHETIQUES DU CONJUGUE DE L'UBIQUITINE ET DE L'HISTONE H2A**
SYNTHETISCHE PEPTIDE DES KONJUGATES VON UBIQUITIN UND HISTON H2A
SYNTHETIC PEPTIDES OF THE CONJUGATE OF UBIQUITINE AND HISTONE H2A

(30) Priorité: 26.04.1989 FR 8905531
(43) Date de publication de la demande: 24.04.1991
(73) Titulaire: PASTEUR SANOFI DIAGNOSTICS, 92430 Marnes La Coquette (FR)
(72) Inventeur: PLAUE, Serge, F-67599 Haguenau (FR); MULLER, Sylviane, F-67000 Strasbourg (FR); VAN REGANMORTEL, Marc, F-67000 Strasbourg (FR)
(74) Mandataire: Polus, Camille
(86) Numéro de dépôt international: FR9000288
(87) Numéro de publication internationale: WO9012806

(56) Documents cités:
- EMBO JOURNAL, vol. 6, no. 4, 1987, IRL Press Ltd., Oxford (GB); A.W. THORNE et al., pp. 1005-1010#

## Description

La présente invention a pour objet des peptides susceptibles d'être reconnus par les anticorps présents dans des fluides biologiques notamment des sérums de patients ou d'animaux atteints de maladies autoimmunes comme le Lupus Erythemateux Disséminé (LED) ou de maladies du systéme nerveux telles que les maladies d'Alzheimer ou de Parkinson.

L'invention concerne également les applications de ces peptides et des compositions les contenant pour le diagnostic in vitro chez l'homme de potentialité de certaines maladies autoimmunes ou du système nerveux, ainsi que leur utilisation à la constitution de trousses ou "kits" de diagnostic.

L'invention concerne en outre les applications de ces peptides à la production de compositions immunogènes et de compositions vaccinantes contre ces maladies.

L'invention concerne enfin les anticorps susceptibles d'être induits in vivo par ces peptides immunogènes ou rendus immunogènes et l'application de ces anticorps et des compositions les contenant pour le diagnostic in vitro chez l'homme atteint de maladies autoimmunes ou du système nerveux, ainsi qu'à la production de médicaments contre ces maladies.

Les protéines de stress sont impliquées dans une variété de maladies telles que les infections non-virales et certaines maladies autoimmunes. Ainsi, S.Muller et al. (Proc. Natl. Acad. Sci. USA 85:8176,1988) ont décrit l'ubiquitine, une protéine de choc thermique contenant 76 résidus d'acides aminés et présente dans toutes les cellules eucaryotes, comme une cible antigénique majeure du Lupus Erythémateux Disséminé (LED) . V.Manetto et al ont montré (Proc. Natl. Acad. Sci. USA 85:4501,1988) que l'ubiquitine est impliquée dans des maladies du système nerveux comme les maladies d'Alzheimer et de Parkinson. B.S.POLLA (Immunol. Today 9:134,1988) a montré que les protéines de choc thermique peuvent jouer un rôle central dans tout les cas d'inflammation où l'augmentation de température est un signe clinique majeur.

Il est admis aujourd'hui que l'ubiquitine peut être présente dans les cellules à la fois de façon libre ou conjuguée à un grand nombre de protéines du noyau, du cytoplasme ou de la membrane ou également associée au réseau de microtubules.

Les conjugués de l'ubiquitine du cytosol sont connus comme étant des médiateurs sélectifs de la dégradation des protéines endommagées ou anormales.

Au niveau du noyau, le rôle des conjugués de l'ubiquitine avec les histones H2A et H2B demeure mal compris. La formation de ces conjugués reléve d'une modification sélective, réversible des histones qui intervient surtout dans les régions activement transcrite de la chromatine et qui peut être impliquée dans la relaxation de la chromatine. L'ubiquitine peut également moduler indirectement la structure de la chromatine en stimulant l'activité de l'enzyme histone déacétylase. Un article de M.Rechsteiner (Ann. Rev. Cell Biol. 3:1,1987) énonce que les conjugués de l'ubiquitine avec les histones du noyau sont dégradés plus lentement que les conjugués de l'ubiquitine du cytosol.

H.Busch et al (Molec. Cell biol. 3:1,1981) et A.W.Thorne et al (EMBO J.6:1005,1987) ont décrit qu'au niveau du noyau l'ubiquitine est conjuguée enzymatiquement avec les histones via une liaison peptidique entre le groupe α-COOH de la glycine C-terminale en position 76 de l'ubiquitine et le groupe ε-NH2 de la chaine latérale de la lysine en position 119 dans l'histone H2A et en position 120 dans l'histone H2B, formant ainsi des molécules branchées.

Dans les cellules de mammiféres, les histones H2A et H2B conjuguées avec l'ubiquitine sont présentes à des taux respectif de 10% et 1%.

S.Muller et al ont décrit (Proc. Natl. Acad. Sci.USA 85:8176,1988) la présence dans les sérums de patients atteints de le Lupus Erythemateux Disséminé, d'anticorps capables de réagir dans un test immunoenzymatique du type ELISA avec l'ubiquitine et avec un fragment synthétique correspondant aux résidus d'acides aminés 22 à 45 de ladite ubiquitine.

Des études plus poussées ont permis à la demanderesse de constater que les sérums de malades atteints de Lupus Erythemateux Disséminé réagissent également dans des techniques immunotransfertblot avec deux polypeptides d'environ 52 et 43 kilodaltons identifiés de manière surprenante comme étant des contaminants dans une préparation commerciale d'ubiquitine.

En utilisant un antisérum spécifique des histones H2A et H2B, la demanderesse a mis en évidence que ces deux polypeptides de 43 et 52 kilodaltons correspondaient respectivement à de l'ubiquitine conjuguée avec l'histone H2A et à un mélange d'ubiquitine conjuguée avec l'histone H2A et avec l'histone H2B.

Les travaux réalisés sur la séquence peptidique de l'ubiquitine et des conjugués de celle-ci avec les histones ont conduit les inventeurs à préparer des peptides synthétiques correspondant à la partie branchée du conjugué de l'ubiquitine avec l'histone H2A, leur permettant de confirmer la présence du conjugué de l'ubiquitine avec l'histone H2A dans les polypeptides de 43 et 52 kilodaltons.

Les antisérums d'animaux immunisés avec ces peptides réagissent spécifiquement avec le conjugué de l'ubiquitine et de l'histone H2A et pas avec l'histone H2A et/ou avec l'ubiquitine libre. En outre, Les sérums de patients atteints de Lupus Erythemateux Disséminé réagissant immunologiquement avec l'ubiquitine réagissent également avec ces peptides.

Ces peptides sont donc applicables au diagnostic du Lupus Erythemateux Disséminé et plus largement à toutes maladies autoimmunes ou du système nerveux dans lesquelles interviennent le conjugué de l'ubiquitine avec l'histone H2A.

Les travaux menés sur ces peptides ont permis de mettre en évidence leur intérêt immunogéne ou à être rendu immunogène afin d'induire in vivo la production d'anticorps susceptible de reconnaître le conjugué de l'ubiquitine avec l'histone H2A, lequel conjugué semble jouer un rôle important dans l'apparition des autoanticorps dans le Lupus Erythemateux Disséminé, d'ou l'utilisation toute indiquée de ces peptides pour la préparation de vaccin contre ce types de maladies.

Pour désigner ci-après les résidus d'acides aminés entrant dans la constitution des peptides selon l'invention, on aura recours à la nomenclature désignant chaque acide aminé naturel par trois lettres, selon le tableau des correspondances suivant :
- Ala: alanine
- Cys: cystéine
- Asp: acide aspartique
- Glu: acide glutamique
- Phe: phénylalanine
- Gly: glycine
- His: histidine
- Ile: isoleucine
- Lys: lysine
- Leu: leucine
- Met: méthionine
- Pro: proline
- Arg: arginine
- Ser: serine
- Thr: thréonine
- Val: valine
- Trp: tryptophane
- Tyr: tyrosine

Les peptides selon l'invention présentent des propriétés immunologiques en commun avec le peptide répondant à la formule suivante :

Gly-Gly-Arg-Leu
Lys-Lys-Thr-Glu (I)

Des peptides préférés selon l'invention répondent à la formule suivante : 
Gly-Gly-Arg-Leu-Y
|
X-Lys-Lys-Thr-Glu-Z (II)
dans laquelle :
. X et Y représentent soit un groupe NH2 libre ou amidé par un ou deux groupes acyle comprenant de 1 à 5 atomes de carbone, soit un groupe peptidique comprenant de 1 à 10 résidus d'acides aminés dont l'acide aminé N-terminal présente un groupe NH2 libre ou amidé comme précédemment.
. Z représente soit un groupe OH libre ou alcoxyle et contenant alors un groupe alcoyle comprenant de 1 à 5 atomes de carbones, soit un groupe peptidique comprenant de 1 à 10 résidus d'acides aminés dont l'acide aminé C-terminal présente on groupe OH libre ou alcoxyle comme précédemment.

Les groupes de 1 à 10 résidus d'acides aminés contenus dans X et/ou Y et/ou Z sont choisis de façon à préserver pour l'essentiel les propriétés immunologiques du peptide de formule (I).

Dans la formule (I), la séquence Gly-Gly-Arg-Leu correspond à la fraction de séquence de l'ubiquitine et la séquence Lys-Lys-Thr-Glu correspond à la fraction de séquence de l'histone H2A, au niveau du branchement entre ces deux composés àans le conjugué de l'ubiquitine avec l'histone H2A. Ainsi, les groupes de 1 à 10 résidus d'acides aminés contenus dans X et/ou Y et/ou Z peuvent notamment correspondre aux résidus d'acides aminés contigüs du peptide de formule (I) dans le conjugué de l'ubiquitine avec l'histone H2A.

Parmi les peptides de formule (II), on préfére ceux pour lesquels :
Y représente soit un groupe NH2 libre, soit un résidu Tyr présentant un groupe NH2 libre, X représente un résidu Cys dont la fonction amine est éventuellement acétylée et Z représente un groupe OH.

Parmi les peptides de formule (II), on préfère également ceux pour lesquels :
.Y représente soit un groupe NH2 libre, soit un résidu Tyr présentant un groupe NH2 libre, X représente l'enchaînement des deux résidus Leu et Pro, le résidu Pro étant lié par une liaison peptidique au résidu Lys contiguë de X dans la formule (II) et la fonction amine du résidu Leu étant éventuellement acétylé et Z représente un groupe OH.

L'invention envisage à titre spécifique les peptides répondant aux formules suivantes : 
Gly-Gly-Arg-Leu-Tyr-NH2
(Ac)Leu-Pro-Lys-Lys-Thr-Glu-OH (III)
dans laquelle la fonction amine du résidu Leu est acétylé (Ac), 
Gly-Gly-Arg-Leu-Tyr-NH2
|
(Ac)Cys-Lys-Lys-Thr-Glu-OH (IV)
dans laquelle la fonction amine du résidu Cys est acétylé (Ac).

Outre les peptides précités, l'invention concerne les peptides modifiés par insertion et/ou délétion et/ou substitution d'un ou plusieurs acides aminés, pour autant que les propriétés antigéniques ou immunogènes desdits peptides ne sont pas modifiées, ainsi que ceux dans lesquels la liaison peptidique (-CO-NH-) est remplacé par exemple par les structures suivantes :
-CO-N(CH3)-, -CH2-CH2-, -CO-CH2-,
ou encore dans lesquels le squelette peptidique présente un ou plusieurs groupes intercalés tels que les groupes -CH2-, -NH-, -O-. La présente invention englobe également les peptides dans lesquels les résidus d'acides aminés présentant un carbone asymétrique sont sous forme D ou L.

Les peptides selon l'invention peuvent être préparés par les techniques classiques de synthèse peptidique en phase solide soit par condensation successive des résidus d'acides aminés dans l'ordre requis, soit par condensation des résidus d'acides aminés sur un fragment préalablement formé et contenant déjà plusieurs acides aminés dans l'ordre approprié ou encore par condensation de plusieurs fragments préalablement préparés, en prenant soin de protéger au préalable toutes les fonctions réactives portées par les résidus d'acides aminés ou les fragments, exceptées les fonctions amine et carboxyle engagées dans la liaison peptidique formée lors de la condensation.

Selon un exemple de préparation d'un peptide selon l'invention, on fixe sur une résine, par l'intermédiaire de son groupe carboxylique le résidu Glu dont la fonction amine est protégée par un groupe terbutyloxycarbonyl, puis après avoir déprotégé la fonction amine en lavant la résine avec de l'acide trifluoroacétique dans du dichlorométhane, on couple dans la dimethylformamide le second résidu d'acide aminé dont la fonction amine est protégée comme précédemment, on fixe ainsi les uns après les autres les résidus d'acides aminés qui vont constituer la portion du peptide selon l'invention correspondant à la fraction de séquence de l'histone H2A. Après déprotection la fonction amine du résidu N-terminal peut être acétylée par action d'un excès d'anhydride acétique en présence de diisopropylethylamine.

Les chaines latérales des acides aminés trifonctionnels doivent être protégées par exemple par les groupes suivants : le cyclohexyle pour l'acide glutamique, le benzyle pour la thréonine, le tosyle pour l'arginine, le paramethylbenzyle pour la cystéine, le 2,6-dichlorobenzyle pour la tyrosine, le fluorenylmethyloxycarbonyl pour l'une des lysines et le 2-chlorobenzyloxycarbonyle pour l'autre lysine.

La chaîne latérale de la lysine sur laquelle se fait le branchement est avantageusement protégée par un groupe fluorenylmethoxycarbonyl, aprés déprotection de celle ci avec un mélange de pipéridine et de dimethylformamide on couple comme précédemment le premier résidu de glycine correspondant à la fraction de séquence de l'ubiquitine, à partir duquel on couple de proche en proche les acides aminés qui vont constituer la chaîne peptidique sur le groupe amine chaque fois déprotégé au préalable, la portion déjà formée restant rattachée à la résine.

Après avoir éliminé tous les groupes protecteurs, le peptide selon l'invention est libéré du support solide par exemple avec de l'acide fluorydrique. Le produit brut est lyophilisé et soumis à une chromatographie en phase liquide à moyenne pression permettant d'obtenir un produit pur à environ 93%, celui-ci sont alors caractérisés par chromatographie en phase liquide à haute pression ainsi que par l'analyse de leur composition en acides aminés et par spectrométrie de masse.

L'analyse de la composition en acides aminés des peptides de formules (III) et (IV) donne les résultats suivants : les valeurs attendues sont entre parenthéses.
- peptide de formule (III) :
   Glu 1,00(1);Gly 2,00(2);Arg 0,95(1);Thr 1,15(1);Pro 0,95(1);Tyr 1,02(1);Leu 1,82(2);Lys 2,11(2)
- peptide de formule (IV) :
   Glu 1,01(1);Gly 1,97(2);Arg 0,97(1);Thr 1,08(1);Tyr 1,03(1);Cys 0,87(1);Leu 1,06(1);Lys 1,87(2)
   La masse moléculaire des peptides de formules (III) et (IV) confirmée par spectrométrie de masse en méthode FAB est la suivante : les valeurs calculées sont entre parenthèses.
- peptide (III) : 1303,8 (1303,5)
- peptide (IV) : 1196,6 (1196,6)

La stabilité des peptides de formules (III) et (IV) a été contrôlée par analyse HPLC; aucune dégradation n'a été constatée après plusieurs mois de stockage à température ambiante et dans le noir, la dimérisation du peptide de formule (IV) du fait de son résidu Cys terminal est inférieure à 9%.

L'invention concerne également les conjugués obtenus par couplage des peptides selon l'invention à des molécules porteuses éventuellement physiologiquement acceptable et non toxique.

Ainsi, les peptides répondant la formule (II) dans laquelle Y représente un résidu Tyr peuvent être avantageusement conjugués à une protéine porteuse au moyen de bis-diazobenzidine. Selon une autre forme de réalisation de l'invention, les peptides répondant à la formule (II) dans laquelle X représente un résidu Cys peuvent être conjugués à une molécule porteuse ou à un. support grâce au groupe thiol.

A titre de molécule porteuse on peut citer des protéines naturelles comme l'anatoxine tétanique, l'albumine ou des sérums albumines.

Les peptides de l'invention possèdent des propriétés antigèniques et peuvent donc être utilisés dans des procédés de diagnostic pour la détermination ou le suivi de patients atteints de maladies autoimmunes ou du système nerveux dans lesquelles le conjugué de l'ubiquitine avec l'histone H2A est impliqué. L'invention concerne également une composition contenant au moins l'un des peptides susceptible d'être reconnu par les autoanticorps présents dans le sérum ou tout autre fluide biologique de patients atteints d'une de ces maladies. La détection du complexe peptide-anticorps in vitro est effectuée par des tests immmunoenzymatiques du type ELISA, d'immunofluorescences, radioimmunologiques ou de radioimmunoprécipitation.

Pour la mise en oeuvre de ces tests, l'invention concerne également les peptides selon l'invention marqués à l'aide d'un marqueur adéquat qui peut être de la biotine ou ses dérivés, une enzyme comme la peroxydase, un marqueur fluorescent comme la fluorescéine, un marqueur radioactif comme un radioisotope, etc...

De tels tests comprennent par exemple les étapes suivantes :
- dépôt d'une quantité déterminée d'une composition contenant un peptide ou un conjugué d'un peptide selon l'invention, dans les puits d'une microplaque de titration ou sur un autre support tel que des billes,
- dépôt dans les puits du liquide biologique à tester ou incubation de celui ci avec les billes,
- aprés incubation et rinçage des microplaques ou des billes, dépôt dans les puits ou incubation avec les billes d'un système de révélation du complexe peptide-anticorps éventuellement formé.

Les tests immunoenzymatiques du types ELISA effectués sur des sérums de patients atteints de Lupus Erythémateux Disséminé ont mis en évidence que 96% d'une population donnée de sérums reconnaissant l'ubiquitine réagissent avec les peptides de formules (IV), à l'inverse seulement 13% des sérums ne réagissant pas avec l'ubiquitine possèdent des autoanticorps pouvant lier le peptide de formule (IV). Sur le groupe de patients atteints de Lupus Erythemateux Disséminé étudié, 51,8% des sérums contiennent des autoanticorps contre l'histone H2A, et 53,8% de ceux-ci réagissent avec le peptide de formule (IV) contre 46,1 qui ne réagissent pas.

L'invention concerne encore les anticorps formés contre les peptides de l'invention. Anticorps qui peuvent être polyclonaux, ou monoclonaux et produits alors par tout hybridome préparé selon les méthodes classiques de fusion cellulaire entre des cellules spléniques d'un animal immunisé contre l'un des peptides de l'invention et des cellules d'une lignée de cellule myélome.

Des antisérums de lapin ont été préparés à partir du peptide de formule (IV) conjugué à de l'ovalbumine, les anticorps obtenus ne réagissent pas avec l'ubiquitine ni avec l'histone H2A lors de tests immunoenzymatiques du type ELISA. Par des techniques d'immunotransfert, ces anticorps ont permis de montrer que les fractions contaminantes de 43 et 52 kilodaltons correspondaient à de conjugués de l'ubiquitine avec l'histone H2A.

En raison de la structure branchée tout à fait particulière des peptides de l'invention, les anticorps préparés à partir de ces peptides constituent des sondes très spécifiques des conjugués de l'ubiquitine et de l'histone H2A incapables de lier l'ubiquitine ou l'histone H2A libres.

Alors qu'il était impossible à ce jour de distinguer l'ubiquitine libre de l'ubiquitine conjuguée avec l'histone H2A, les anticorps selon l'invention permettent d'identifier sans équivoque le conjugué.

Par ailleurs, les anticorps formés contre les peptides de l'invention et les autoanticorps de patients réagissant avec lesdits peptides et obtenu après chromatographie d'affinité peuvent être utilisés pour préparer des anticorps anti-idiotypes constituant en partie une copie exacte du peptide antigènique initial et donc capables de se lier aux autoanticorps observés dans les maladies autoimmunes.

La présente invention concerne également ces anticorps anti-idiotypes et les compositions les contenant ainsi que leur application pour le diagnostic in vitro chez l'homme de la présence d'autoanticorps et la production de médicament contre les maladies autoimmunes.

L'invention concerne également des compositions immunogènes pour la production de vaccins dont le principe actif est constitué par au moins un peptide ou un anticorps anti-idiotype selon l'invention, éventuellement conjugué à une molécule porteuse, induisant la production d'anticorps contre lesdits peptides et qui sont capables d'interférés avec la pathologie et/ou les manifestations cliniques des maladies autoimmunes. Les compositions pharmaceutiques, selon l'invention, utilisables comme vaccin sont constituées par des solutions ou suspensions injectables ou administrables par d'autres voies pouvant être administrées à des doses situées entre 10 µg/kg et 100mg/kg de peptides selon l'invention.

## Revendications

1. Peptides caractérisés en ce qu'ils présentent des propriétés immunologiques en commun avec le peptide répondant à la formule suivante :

    Gly-Gly-Arg-Leu
     |
Lys-Lys-Thr-Glu                    (I)

2. Peptides selon la revendication 1, caractérisés en ce qu'ils répondent à la formule : 
      Gly-Gly-Arg-Leu-Y
       |
X-Lys-Lys-Thr-Glu-Z          (II)
 dans laquelle :
.X et Y représentent soit un groupe NH2 libre ou amidé par un ou deux groupes acyle comprenant de 1 à 5 atomes de carbone, soit un groupe peptidique comprenant de 1 à 10 résidus d'acides aminés dont l'acide aminé N-terminal présente un groupe NH2 libre ou amidé comme précédemment.
.Z représente soit un groupe OH libre ou alcoxyle et contenant alors un groupe alcoyle comprenant de 1 à 5 atomes de carbones, soit un groupe peptidique comprenant de 1 à 10 résidus d'acides aminés dont l'acide aminé C-terminal présente on groupe OH libre ou alcoxyle comme précédemment.

3. Peptides selon la revendication 2, caractérisés en ce que dans la formule (II) :
Y représente soit un groupe NH2 libre, soit un résidu Tyr présentant un groupe NH2 libre, X représente l'enchaînement des deux résidus Leu et Pro, le résidu Pro étant lié par une liaison peptidique au résidu Lys contiguë de X dans la formule (II) et la fonction amine du résidu Leu étant éventuellement acétylé et Z représente un groupe OH.

4. peptide selon la revendication 3, caractérisé en ce qu'il répond à la formule suivante : 
Gly-Gly-Arg-Leu-Tyr-NH2
(Ac)Leu-Pro-Lys-Lys-Thr-Glu-OH (III)
 dans laquelle la fonction amine du résidu Leu est acétylée(Ac).

5. Peptides selon la revendication 2, caractérisés en ce que dans la formule (II) :
.Y représente soit un groupe NH2 libre, soit un résidu Tyr présentant un groupe NH2 libre, X représente un résidu Cys dont la fonction amine est éventuellement acétylée et Z représente un groupe OH.

6. Peptide selon la revendication 5 caractérisé en ce qu'il répond à la formule suivante : 
            Gly-Gly-Arg-Leu-Tyr-NH2
            |
(Ac)Cys-Lys-Lys-Thr-Glu-OH                    (IV)
 dans laquelle la fonction amine du résidu Cys est acétylée(Ac).

7. Anticorps formés contre un peptide selon l'une quelconque des revendications 1 à 6, caractérisés en ce qu'ils reconnaissent, de manière spécifique, le conjugué de l'ubiquitine avec l'histone H2A.

8. Anticorps anti-idiotypes formés contre un anticorps selon la revendication 7, caractérisé en ce qu'ils reconnaissent les autoanticorps présents dans des fluides biologiques, notamment des sérums, de patients atteints d'une maladie autoimmune.

9. Anticorps anti-idiotypes formés contre un autoanticorps réagissant avec un peptide selon l'une quelconque des revendications 1 à 6 caractérisé en ce qu'ils reconnaissent les autoanticorps présents dans des fluides biologiques, notamment des sérums, de patients atteints d'une maladie autoimmune.

10. Composition antigénique ccntenant au moins un peptide selon l'une quelconque des revendications 1 à 6, caractérisée en ce qu'elle réagit de manière spécifique avec les autoanticorps présents dans des fluides biologiques humains, notamment des sérums, de patients atteints d'une maladie autoimmune.

11. Composition immunogène contenant au moins un peptide selon l'une quelconque des revendications 1 à 6 ou un conjugué de ce peptide avec une molécule porteuse, caractérisée en ce quelle induit la production d'anticorps capables de reconnaître le conjugué de l'ubiquitine avec l'histone H2A.

12. Procédé de diagnostic in vitro de la présence d'autoanticorps dans un fluide biologique, caractérisé en ce qu'il comprend au moins les étapes suivantes :
- la mise en contact de ce fluide biologique avec au moins un peptide selon l'une des revendications 1 à 5 ou un conjugué de ces peptides avec une molécule porteuse ou encore un anticorps anti-idiotype selon la revendication 8,
- la détection de la présence d'un complexe antigéne-anticorps ou d'un complexe anticorps-anticorps anti-idiotype par des méthodes physiques ou chimiques, dans ledit fluide biologique.

## Patentansprüche

1. Peptide, **dadurch gekennzeichnet,** daß sie immunologische Eigenschaften besitzen, die gemeinsam sind mit denen des Peptids der folgenden Formel:

Gly-Gly-Arg-Leu
|
Lys-Lys-Thr-Glu (I)

2. Peptide nach Anspruch 1, **dadurch gekennzeichnet**, daß sie der Formel: 
Gly-Gly-Arg-Leu-Y
|
X-Lys-Lys-Thr-Glu-Z (II)
 entsprechen, in der:
- X und Y entweder eine freie oder durch eine oder zwei acyl gruppen mit 1 bis 5 Kohlenstoffatomen amidierte NH₂-Gruppe oder eine Peptidgruppe mit 1 bis 10 Aminosäureresten, deren N-terminale Aminosäure eine, wie oben angegeben, freie oder alkylierte NH₂-Gruppe aufweist und
- Z entweder eine freie oder alkoxylierte und damit eine Alkylgruppe mit 1 bis 5 Kohlenstoffatomen aufweisende OH-Gruppe oder eine Peptidgruppe, die 1 bis 10 Aminosäurereste aufweist, deren C-terminale Aminosäure eine wie oben definierte freie oder alkoxylierte OH-Gruppe aufweist,
bedeuten.

3. Peptide nach Anspruch 2, **dadurch gekennzeichnet**, daß in der Formel (II):
Y entweder eine freie NH₂-Gruppe oder einen eine freie NH₂-Gruppe aufweisenden Rest Tyr, X eine Kette aus den beiden Resten Leu und Pro, wobei der Rest Pro durch eine Peptidbindung an den an X in der Formel (II) angrenzenden Rest Lys gebunden ist und die Aminogruppe des Rests Leu gegebenenfalls acetyliert ist und Z eine OH-Gruppe bedeuten.

4. Peptid nach Anspruch 3, **dadurch gekennzeichnet,** daß es der folgenden Formel entspricht: 
Gly-Gly-Arg-Leu-Tyr-NH2
|
(Ac)Leu-Pro-Lys-Lys-Thr-Glu-OH (III)
 in der die Aminogruppe des Rests Leu acetyliert ist (Ac).

5. Peptide nach Anspruch 2, **dadurch gekennzeichnet,** daß in der Formel (II):
- Y entweder eine freie NH₂-Gruppe oder einen eine freie NH₂-Gruppe aufweisenden Rest Tyr, X einen Rest Cys, dessen Aminogruppe gegebenenfalls acetyliert ist, und Z eine OH-Gruppe bedeuten.

6. Peptid nach Anspruch 5, **dadurch gekennzeichnet**, daß es der folgenden Formel entspricht: 
Gly-Gly-Arg-Leu-Tyr-NH2
|
(Ac)Cys-Lys-Lys-Thr-Glu-OH
(IV)
 in der die Aminogruppe des Rests Cys acetyliert ist (Ac).

7. Antikörper, gebildet gegen ein Peptid nach einem der Ansprüche 1 bis 6, **dadurch gekennzeichnet,** daß sie in spezifischer Weise das Konjugat aus Ubiquitin und Histon H2A erkennen.

8. Anti-idiotype Antikörper, gebildet gegen einen Antikörper nach Anspruch 7, **dadurch gekennzeichnet,** daß sie Autoantikörper erkennen, die in biologischen Flüssigkeiten, insbesondere Seren, von Patienten vorhanden sind, die an einer Autoimmunkrankheit leiden.

9. Anti-idiotype Antikörper, gebildet gegen einen Autoantikörper, der mit einem Peptid nach einem der Ansprüche 1 bis 6 reagiert, **dadurch gekennzeich****net,** daß sie Autoantikörper erkennen, die in biologischen Flüssigkeiten, insbesondere Seren von Patienten enthalten sind, die an einer Autoimmunkrankheit leiden.

10. Antigene Zubereitung enthaltend mindestens ein Peptid nach einem der Ansprüche 1 bis 6, **dadurch gekennzeichnet,** daß sie in spezifischer Weise mit den in menschlichen biologischen Flüssigkeiten, insbesondere Seren von Patienten, die an einer Autoimmunkrankheit leiden, vorhandenen Autoantikörpern reagieren.

11. Immunogene Zubereitung enthaltend mindestens ein Peptid nach einem der Ansprüche 1 bis 6 oder ein Konjugat dieses Peptids mit einem Trägermolekül, **dadurch gekennzeichnet,** daß sie die Bildung von Antikörpern induziert, die dazu in der Lage sind, das Konjugat von Ubiquitin mit Histon H2A zu erkennen.

12. Verfahren zur in vitro-Diagnose der Anwesenheit von Autoantikörpern in einer biologischen Flüssigkeit, **dadurch gekennzeichnet,** daß es mindestens die folgenden Stufen umfaßt:
- Inkontaktbringen dieser biologischen Flüssigkeit mit mindestens einem Peptid nach einem der Ansprüche 1 bis 5 oder einem Konjugat dieser Peptide mit einem Trägermolekül oder mit einem anti-idiotypen Antikörper nach Anspruch 8,
- Nachweis der Anwesenheit eines Antigen-Antikörper-Komplexes oder des antikörper-anti-idiotypen Antikörper-Komplexes mit Hilfe von physikalischen oder chemischen Methoden in der genannten biologischen Flüssigkeit.

## Claims

1. Peptides characterised in that they have immunological properties in common with the peptide corresponding to the following formula:

Gly-Gly-Arg-Leu
|
Lys-Lys-Thr-Glu (I)

2. Peptides according to claim 1 characterised in that they correspond to the formula: 
      Gly-Gly-Arg-Leu-Y
      |
X-Lys-Lys-Thr-Glu-Z                    (II)
 in which:
X and Y represent either a free NH₂ group or an NH₂ group amidified by one or two acyl groups containing from 1 to 5 carbon atoms, or a peptide group containing from 1 to 10 amino acid residues in which the N-terminal amino acid is an NH₂ group, free or amidified as above;
Z represents either a free OH group or an alkoxyl group which includes an alkyl group containing from 1 to 5 carbon atoms, or a peptide group containing from 1 to 10 amino acid residues in which the C-terminal amino acid has a free OH group or alkoxyl group as above.

3. Peptides according to claim 2 characterised in that in formula (II):
Y represents either a free NH₂ group or a Tyr residue containing a free NH₂ group, X represents the two residues Leu and Pro linked, the Pro residue being bonded by a peptide bond to the Lys residue adjacent to X in formula (II) and the amino group of the Leu residue being optionally acetylated, and Z represents an OH group.

4. A peptide according to claim 3 characterised in that it corresponds to the following formula: 
Gly-Gly-Arg-Leu-Tyr-NH2
|
(Ac)Leu-Pro-Lys-Lys-Thr-Glu-OH (III)
 in which the amino group of the Leu residue is acetylated (Ac).

5. Peptides according to claim 2 characterised in that in formula (II):
Y represents either a free NH₂ group or a Tyr residue containing a free NH₂ group, X represents a Cys residue in which the amino group is optionally acetylated and Z represents an OH group.

6. Peptide according to claim 5 characterised in that it corresponds to the following formula: 
Gly-Gly-Arg-Leu-Tyr-NH2
(Ac)Cys-Lys-Lys-Thr-Glu-OH (IV)
 in which the amino group of the Cys residue is acetylated (Ac).

7. Antibodies induced by a peptide according to any one of claims 1 to 6 characterised in that they specifically bind to the ubiquitin-H2A histone conjugate.

8. Anti-idiotype antibodies induced by an antibody according to claim 7 characterised in that they bind to the auto-antibodies present in the biological fluids, notably the serums, of patients afflicted by an autoimmune disease.

9. Anti-idiotype antibodies induced by an auto-antibody reagent with a peptide according to any one of claims 1 to 6 characterised in that they bind to auto-antibodies present in biological fluids, notably the serums, of patients afflicted by an autoimmune disease.

10. An antigenic composition comprising at least one peptide according to any one of claims 1 to 6 characterised in that it specifically binds with the auto-antibodies present in human biological fluids, notably the serums, of patients afflicted by an autoimmune disease.

11. An immunogenic composition comprising at least one peptide according to any one of claims 1 to 6 or a conjugate of said peptide with a carrier molecule characterised in that it induces the production of antibodies capable of binding to ubiquitin-H2A histone conjugate.

12. An in vitro process for the diagnosis of the presence of auto-antibodies in a biological fluid characterised in that it comprises at least the following steps:
- placing said biological fluid in contact with at least one peptide according to one of claims 1 to 5 or a conjugate of said peptides with a carrier molecule or an anti-idiotype antibody according to claim 8;
- the detection of the presence of an antigen-antibody complex or of an antibody-anti-idiotype antibody complex by physical or chemical methods in said biological fluid.
